# EUROPÄISCHE PATENTSCHRIFT

(11) **EP 2 723 374 B1**
(45) Veröffentlichungstag und Bekanntmachung des Hinweises auf die Patenterteilung: **06.09.2017**
(21) Anmeldenummer: 12740908.4
(22) Anmeldetag: 27.06.2012
(51) Int. Cl.: A61K 39/39, A61K 39/29, C07K 14/42, A61K 39/12

(54) **REKOMBINANTES MISTELLEKTIN UND DESSEN VERWENDUNG ALS ADJUVANS**
RECOMBINANT MISTLETOE LECTIN AND USE THEREOF AS AN ADJUVANT
LECTINE DU GUI DE RECOMBINAISON ET SON UTILISATION COMME ADJUVANT

(30) Priorität: 27.06.2011 DE 102011118023
(43) Veröffentlichungstag der Anmeldung: 30.04.2014
(73) Patentinhaber: Melema Pharma GmbH, 20148 Hamburg (DE)
(72) Erfinder: WITTHOHN, Klaus, 51491 Overath (DE); LENTZEN, Hans, 51503 Rösrath (DE)
(74) Vertreter: Simandi, Claus
(86) Internationale Anmeldenummer: PCT/EP2012/062521
(87) Internationale Veröffentlichungsnummer: WO 2013/000980

(56) Entgegenhaltungen:
- EP-A1- 0 751 221
- EP-A2- 1 074 560
- WO-A1-01/34193
- DE-A1- 19 804 210
- SONG ET AL: "Intranasal immunization with influenza virus and Korean mistletoe lectin C (KML-C) induces heterosubtypic immunity in mice", VACCINE, ELSEVIER LTD, GB, Bd. 25, Nr. 34, 26. Juli 2007 (2007-07-26) , Seiten 6359-6366, XP022169848, ISSN: 0264-410X, DOI: 10.1016/J.VACCINE.2007.06.030
- PARK H J ET AL: "TLR4-mediated activation of mouse macrophages by Korean mistletoe lectin-C (KML-C)", BIOCHEMICAL AND BIOPHYSICAL RESEARCH COMMUNICATIONS, ACADEMIC PRESS INC. ORLANDO, FL, US, Bd. 396, Nr. 3, 4. Juni 2010 (2010-06-04), Seiten 721-725, XP027072220, ISSN: 0006-291X, DOI: 10.1016/J.BBRC.2010.04.169 [gefunden am 2010-06-03]
- LAVELLE E C ET AL: "The identification of plant lectins with mucosal adjuvant activity", IMMUNOLOGY, BLACKWELL PUBLISHING, OXFORD, GB, Bd. 102, Nr. 1, 1. Januar 2001 (2001-01-01), Seiten 77-86, XP002165133, ISSN: 0019-2805, DOI: 10.1046/J.1365-2567.2001.01157.X
- LAVELLE ET AL: "Mistletoe lectins enhance immune responses to intranasally co-administered herpes simplex virus glycoprotein D2.", IMMUNOLOGY, Bd. 107, Nr. 2, 1. Oktober 2002 (2002-10-01), Seiten 268-274, XP55029875, ISSN: 0019-2805
- ELSÄSSER-BEILE U ET AL: "Biological effects of natural and recombinant mistletoe lectin and an aqueous mistletoe extract on human monocytes and lymphocytes in vitro.", JOURNAL OF CLINICAL LABORATORY ANALYSIS 2000 LNKD- PUBMED:11138605, Bd. 14, Nr. 6, 2000, Seiten 255-259, XP009163386, ISSN: 0887-8013

## Beschreibung

Die Erfindung betrifft Vakzine, die Antigene und ein Adjuvans umfassen, sowie die Verwendung des Adjuvans, wobei das Adjuvans aus einem rekombinanten Mistellektin ausgewählt ist.

Bei vielen Krankheiten sind Vakzine nur teilweise oder gar nicht wirksam. Die Forschung über schützende Immunität und Adjuvantien, die kräftige Immunantworten generieren, kann helfen, wirksame Vakzine gegen Phatogene zu generieren (McKee AS, MacLeod MKL, Kappler JW et al. 2010 BMC Biology; 8: 37 - 46). Vakzine sind Impfstoffe aus lebenden, attenuierten (d. h. in ihrer Virulenz abgeschwächten) bzw. inaktivierten Krankheitserregern oder aus inaktivierten (entgifteten) Toxinen bzw. Toxoiden von Erregern oder Teilstücken der Oberflächenstruktur von Erregern. Zur Zeit werden formal drei Haupttypen von Vakzinen unterschieden: a.) Attenuierte Lebendimpfstoffe, die aus einem Virus oder einem Bakterium bestehen, das ähnlich aber weniger pathogen ist als das tatsächliche Pathogen; b.) inaktivierte Vakzine, die hitzeinaktivierte oder chemisch inaktivierte Partikel von dem Pathogen sind; oder c.) Subunit-Vakzine, die aus Komponenten des Pathogens hergestellt sind.

Vakzine enthalten neben einem solchen Antigen, welches das Ziel der adaptiven Immunantwort ist, zumeist entweder Pathogen-assoziierte molekulare Muster (PAMPs) oder andere Substanzen, welche die adaptive Antwort verstärken oder beeinflussen. Diese Substanzen sind als Adjuvantien beschrieben (McKee et al 2010 (supra)).

Ein bekanntes Adjuvans, welches eine historisch lange Verwendung in humanen Vakzinen aufweist, ist Aluminiumsalz (auch als Alaun bezeichnet). Proteine (Antigene) vom Pathogen werden auf das Aluminiumsalz adsorbiert, wobei eine Suspension erzeugt wird, die intramuskulär injiziert wird.

Die Hepatitis B ist eine Infektionskrankheit der Leber mit dem Hepatitis-B-Virus (HBV), die häufig akut (90 %), gelegentlich auch chronisch verläuft. Mit etwa 350 Millionen chronisch infizierten Menschen ist die Hepatitis B weltweit die häufigste Virusinfektion. Auf Basis der chronischen Leberentzündung kann eine Leberzirrhose, sowie ein Leberzellkarzinom entstehen. Die Therapie einer chronischen Hepatitis B ist schwierig, daher ist die vorbeugende Impfung die wichtigste Maßnahme zur Vermeidung der Infektion und Verminderung der Virusträgerzahl.

Es sind verschiedene Hepatitis-B-Impfstoffe kommerziell erhältlich, wie z.B. Engerix-B von GlaxoSmithKline. Bei diesem Impfstoff wird Aluminiumhydroxid als Adjuvans verwendet und das Hepatitis B Oberflächenantigen HBsAg wird durch rekombinante DNA-Technologie in Hefezellen (Saccharomyces cerevisiae) hergestellt.

Bei einer Krebsbehandlung mittels Vakzinierung können die Vakzine therapeutisch oder prophylaktisch sein. Adjuvantien werden verabreicht, um die Wirksamkeit von prophylaktischen oder therapeutischen Vakzinen, d.h. eine starke und andauernde Immunantwort hervorzurufen, zu verstärken. Insbesondere besteht ein Bedarf an Subunit-Vakzinen. Subunit-Vakzine bestehen aus gereinigten Antigenen, die von Lymphozyten spezifisch erkannt werden. Obwohl sie sicherer als Gesamtorganismus-Vakzine sind, sind sie alleine nicht in der Lage, das Immunsystem optimal zu aktivieren, da ihnen intrinsische PAMPs fehlen (McKee et al 2010 (supra)). Adjuvantien können die Balance der induzierten antikörper- und zellvermittelten Immunität beeinflussen. Bei der Verwendung solcher Substanzen sind daher eine geringere Dosierung eines Antigens und eine geringere Zahl an Injektionen notwendig (Salk JE, Laurent AM & Bailey ML 1951 Am J Public Health Nations Health 41: 669-77).

Adjuvantien werden zudem in zwei Kategorien unterteilt: Trägersysteme und "immune potentiators", d.h. Immunverstärker (Pashine A, Valiante NM & Ulmer JB 2005 Nature medicine 11: 63-68, Pichichero ME 2008 Human vaccines 4(4): 262 270). Träger-basierte Adjuvantien (Aluminium) erhöhen die Interaktion zwischen Vakzinkomponenten und Schlüsselzellen des Immunsystems.
Immunverstärker aktivieren direkt antigenpräsentierende Zellen (APCs) und angeborene Immunantworten durch Verwendung spezifischer Rezeptoren [z.B. Toll-Like-Rezeptoren (TLRs)] (Pashine et al. (supra), O'Hagan DT & Valiante NM 2003 Nature reviews 2: 727-735).

Seit 1970 sind viele Arten von Vakzin-Adjuvantien verfügbar, aber bis heute sind nur wenige zur Verwendung bei Menschen zugelassen (O'Hagan DT & De Gregorio E 2009 Drug Discov Today 14(11-12): 541-551).

In der letzten Zeit bieten neue Adjuvantien, wie beispielsweise Toll-Like-Rezeptor (TLR)-Agonisten und neue teilchenförmige Trägersysteme, wie peptidbasierte Vakzine eine Option für die moderne Immuntherapie.

Neue Adjuvantien sind jedoch weiterhin notwendig, die die folgenden Eigenschaften aufweisen sollen (Brunner R, Jensen-Jarolim E & Pali-Schöll I 2010 Immunol Lett 128(1): 29-35):
a.) Verstärkung von Th17-Zellen. Diese Adjuvantien sind durch die Produktion von IL-17 charakterisiert. Sie sind ein wichtiger Modulator bei Entzündungen und CD4+ T-Zellerinnerung.
b.) Induzieren starker zellulärer Antworten, einschließlich T-Helfer (Th) 1-Zellen und zytotoxischen T-Lymphozyten (CTLs) zusätzlich zu Antikörpern (Guy B 2007 Nat Rev Microbiol 5: 505-517).

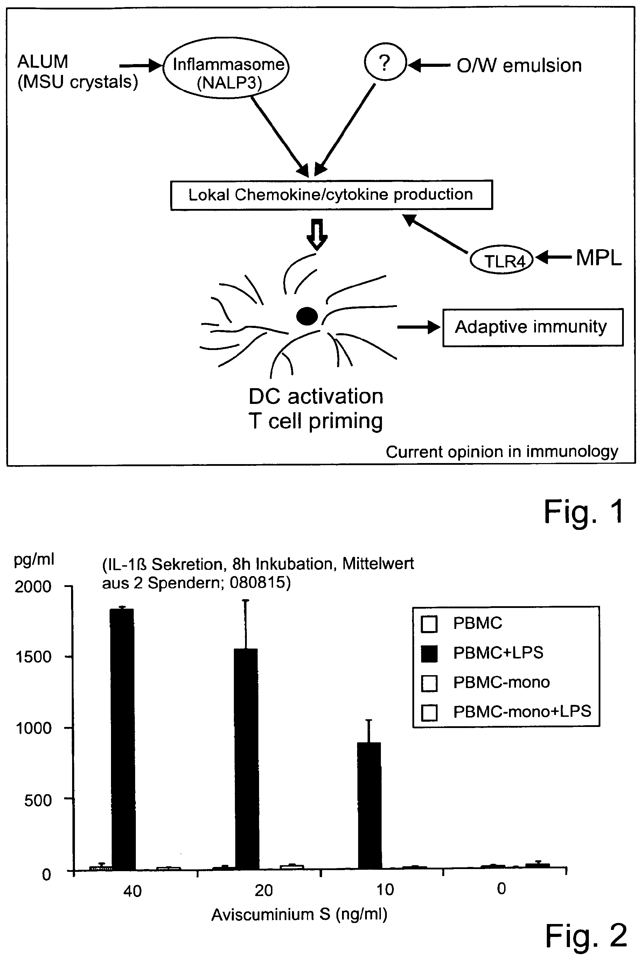

Im Allgemeinen haben Adjuvantien verschiedene Wirkmechanismen (Fig. 1). Nichtspezifische Adjuvantien (z.B. Aluminumhydroxid) verstärken die Antigenpräsentation durch Aktivierung des Inflammasom-Pathway, welcher durch eine Sekretion von IL-1ß (Lamine Mbow M, De Gregorio E, Valianteal NM et al. 2010 Current Opinion in Immunology 2010, 22: 411-416) charakterisiert ist. Aluminium bildet ein Depot an der Injektionsstelle, welches zu einer hohen lokalen Antigenkonzentration führt und daher die Aufnahme durch antigenpräsentierende Zellen (APCs) verbessert (HogenEsch H 2002 Vaccine 20 (Suppl 3): 34-39). Ferner kann die Antigenerkennung mittels direkter Stimulation von Immunzellen beschleunigt werden (Gupta RK, Rost BE, Relyveld E et al 1995 Pharm Biotechnol 6: 229-248). Diese sogenannten Typ-B-Adjuvantien interagieren mit APCs und Antigenen in einer unspezifischen Weise und ihre Wirkung basiert auf einer Verstärkung der antigenpräsentierenden MHC-Moleküle.

Im Gegensatz zu Typ-B-Adjuvantien haben Typ-A-Adjuvantien einen spezifischen Wirkmechanismus. Die meisten der kürzlich entwickelten Typ-A-Adjuvantien, wie z.B.

Monophosphoryl-Lipid A (MPL), sind spezifische Agonisten für TLRs. Sie wirken primär auf TLR, und wirken indirekt mittels Aktivierung von APCs und Auslösen der Sekretion von Zytokinen, wie beispielsweise IL-12. Außerdem können TLR-Agonisten auf MHC-Rezeptoren mittels wirksamer Präsentation der verabreichten Antigene wirken (Guy B 2007 (supra)).

Ferner sind Typ-C-Adjuvantien beschrieben. Ihre Funktionsweise basiert auf einer Verstärkung von MHC-Rezeptoren durch Interaktion mit co-stimulatorischen Molekülen auf APCs. Es gibt Bestrebungen die Typ-C-Adjuvantien in die klinische Anwendung einzuführen. Ein bekanntes Beispiel ist TGN1412, ein neuer superagonistischer anti-CD28 monoklonaler Antikörper, welcher direkt T-Zellen stimuliert, wobei jedoch in einer Studie kardiovaskulärer Schock und akutes Lungenversagen, infolge eines Zytokinensturms aufgetreten sind (Suntharalingam G, Perry MR, Ward S et al. 2006 N Engl J Med 355: 1018-1028).
Es besteht zum Stand der Technik ein hohes Bedürfnis an neuen geeigneten Adjuvantien.

Pflanzliche Mistelextrakte werden seit Jahrhunderten therapeutisch genutzt. Insbesondere in der Krebstherapie sind Mistelpräparate mit unterschiedlichem Erfolg eingesetzt worden (Bocci V 1993 J Biol Regulators and Homeostatic Agents 7(1): 1 - 6; Gabius H-J, Gabius S, Joshi S S et al. 1993 Planta Med 60: 2 - 7; Gabius H-J & Gabius S 1994 PZ 139: 9 - 16; Ganguly C & Das S 1994 Chemotherapy 40: 272 - 278, Hajto T, Hostanska K, Gabius H_J 1989 Cancer Res 49: 4803 - 4808, Hajto T, Hostanska K, Frei K et al. 1990 Cancer Res. 50: 3322 - 3326). Es zeigte sich, dass die therapeutischen Effekte insbesondere durch sogenannte Mistellektine (Viscumine, Viscum album Agglutinine, VAA) vermittelt werden. Den Mistellektinen wird dabei neben einer zytotoxischen Wirkung auch eine unspezifische Immunstimulation zugesprochen, deren positive Effekte zur Therapie von Tumorpatienten genutzt werden. Verschiedene Untersuchungen mit Mistellektinen in vitro (Hajto et al., 1990 (supra); Männel D N, Becker H, Gundt A et al. 1991 Cancer Immunol Immunother 33: 177 - 182; Beuth J, Ko K L, Tunggal L et al. 1993 Drug Res 43: 166 - 169) und in vivo (Hajto T 1986 Oncology 43 suppl 1: 51 - 65; Hajto et al., 1989 (supra), Beuth J, Ko H L, Gabius H-J et al. 1991 In Vivo 5: 29 - 32; Beuth J, Ko H L, Gabius H-J et al. 1992 J Clin Invest 70: 658 - 661), sowie klinische Studien (Beuth et al., 1992 (supra)) zeigten eine erhöhte Freisetzung von inflammatorischen Zytokinen (TNF-alpha, IL-1, IL-6) und eine Aktivierung von zellulären Komponenten des Immunsystems (TH -Zellen, NK-Zellen).

Durch Analysen des Mistelextraktes konnten bisher drei Mistellektine (ML-I, ML-II, ML-III) mit unterschiedlichen Molekulargewichten und Zuckerbindungsspezifitäten identifiziert werden. Es konnte gezeigt werden, dass der immunstimulierende Effekt des Mistelextrakts auf das ML-I zurückzuführen ist. Das ML-I-Lektin besteht aus jeweils zwei glykosylierten A- und B-Ketten (MLA bzw. MLB). Die A-Kette ist für eine enzymatische Inaktivierung von Ribosomen (Endo Y, Tsurugi K & Franz H 1988 FEBS Lett 231: 378 - 380) verantwortlich, während die B-Kette bei der Carbohydratbindung beteiligt ist. Die beiden Ketten sind durch Disulfidbrücken miteinander verknüpft. Die resultierenden Mistellektin-Monomere können sich unter Ausbildung von nicht kovalenten Bindungen zu Dimeren zusammenlagern.

Es ist möglich, das biologisch aktive Mistellektin auch rekombinant herzustellen. EP 0751221 beschreibt die Reindarstellung von Mistellektin-Polypeptiden als strukturell homogene Substanz, wobei ausgehend von den Gensequenzen des Mistellektins rekombinante, hochreine Einzelketten (A-Kette, B-Kette) hergestellt werden, die *in vitro* reassoziiert werden können und so ein rekombinantes Mistellektin-Holoprotein ergeben, das proteinchemisch, enzymatisch und strukturell besonders vorteilhaft homogen ist, sogenanntes "Aviscuminum". Gemäß EP 0751221 ist das rekombinante Mistellektin-Polypeptid sowohl als Holoprotein, als Teilkette und in Form von Subfragmenten für therapeutische Zwecke geeignet und erfindungsgemäß umfasst. Das biologisch aktive Mistellektin kann rekombinant in *E. coli* hergestellt werden und wird neben Aviscuminum auch als "rViscumin" oder "rML" bezeichnet (Eck J, Langer, M, Möckel, B et al 1999; Eur J Biochem 264: 775-784).

In EP 0751221 wird zwar erwähnt, dass rekombinante Mistellektine für die Behandlung von Infektionserkrankungen denkbar wären, jedoch werden keinerlei Hinweise offenbart, dass rekombinantes Mistellektin als Adjuvans bzw. in einem Vakzin verwendet werden kann.

Weiterhin beschreiben Lavelle et al (Lavelle EC, Grant G, Pusztal A et al. 2002. Immunology 107: 268-274) den Einsatz von pflanzlichen Lektinen als Adjuvans, welche mucosal durch Inhalation von 1.000 ng verabreicht werden. Weiterhin offenbart Lavelle, dass eine Dosis von 1.000 ng / Maus sich negativ auf das Gewicht der Tiere und ihr Überleben auswirkt, also toxisch ist.

Song et al (Song SK, Moldoveanu Z, Nguyen HN et al. 2007 Vaccine 25: 6359-6366) offenbaren die Eignung von koreanischem Mistellektin als Adjuvans, welche mucosal verabreicht werden. DE 19894210 (EP 1 051 495) beschreibt eine Zusammensetzung, welche ein Mistel-Lektin in Verbindung mit mindestens einem weiteren Antigen enthält und zur Herstellung eines Medikamentes dient. Besagtes Medikament vermag die Immunreaktion gegen das weitere Antigen zu verstärken, d.h. eine Aktivierung von T-Lymphocyten und Lymphokin-produzierenden Makrophagen hervorrufen. Allerdings sind pflanzliche Mistellektine inhomogen (Soler MH, Stoeva S, Schwamborn C et al. 1996 FEBS Letter 399: 153-157, Soler HS, Stoeva S, Voelter W 1998 Biochem Biophys Res Comm 246: 596-601) und untereinander in der Wirkung uneinheitlich bis verschieden (EP 1051495 B1), und nicht per se als Adjuvans oder als Immunmodulator wirksam. Von daher ist z.B. das koreanische Mistellektin zwar den RIP II Proteinen zuzuordnen, weist jedoch starke strukturelle Unterschiede in der Struktur und Konformation gegenüber den hier in Rede stehenden rekombinanten Mistellektinen auf (Kang TB, Song SK, Yoon TJ et al. 2007 J Biochem Mol Biol 40(6): 959-965). Nachteilig ist, dass keine exakte Dosiseinstellung möglich ist und pflanzliche Mistellektine Verunreinigungen aufweisen. Weiterhin zeigen die pflanzlichen Mistellektinen Unterschiede in der Glycolisierung, die die Wirksamkeit als Adjuvans beeinflussen (insbesondere Kinetik, etc.) können.

Die erfindungsgemäßen rekombinanten Mistellektine weisen vorteilhaft keine solche Glycolisierung auf.

Es ist wünschenswert, die Immunogenität von Antigenen durch die Verwendung von Adjuvantien zu erhöhen, um eine effiziente und verbesserte immunologische Antwort in einem Wirt oder Patienten zu erhalten. Es besteht ferner ein hoher Bedarf an sicheren und wirksamen Adjuvantien, die die Wirkung von Vakzinen erhöhen und leicht zu verwenden sind.

Die Aufgabe der vorliegenden Erfindung besteht darin, ein Vakzin oder Adjuvans bereitzustellen, mit der die Immunantwort eines Wirts oder Patienten gegen Antigene erhöht werden kann.

Die Aufgabe wird durch die Bereitstellung eines Vakzins oder Adjuvans gelöst, welches rekombinantes Mistellektin enthält und zwar bestehend aus den Sequenzen SEQ ID No. 1 und SEQ ID No. 4

Es wurde überraschender Weise festgestellt, dass ein solches rekombinantes Mistellektin als potentielles Adjuvans dienen kann, wenn es zusammen mit einem Antigen verabreicht wird.

Daher betrifft die Erfindung ein Adjuvans enthaltend mindestens ein solches rekombinantes Mistellektin (nachstehend erfindungsgemäßes Adjuvans).

Ferner betrifft die Erfindung ein Vakzin enthaltend ein Antigen samt einem erfindungsgemäßen Adjuvans (nachstehend erfindungsgemäßes Vakzin).

Daher betrifft die Erfindung ebenfalls ein Arzneimittel oder Zusammensetzung enthaltend ein erfindungsgemäßes Vakzin und ggfs. weitere Hilfs- und Zusatzstoffe und / oder einen pharmazeutisch verträglichen Träger oder ein pharmazeutisch verträgliches Verdünnungsmittel.

Die Aufgabe wird durch die Bereitstellung eines Adjuvans oder einem Vakzin gelöst, wobei diese rekombinante Mistellektine enthalten und die rekombinanten Mistellektine die folgenden Aminosäuresequenzen umfassen:
Das rekombinante Mistellektin-Polypeptid der Mistellektin-A Kette umfasst die folgenden Sequenzen: SEQ ID No. 1, einschließlich deren Isoformen oder ein funktionelles Fragment davon.

Das rekombinante Mistellektin-Polypeptid der Mistellektin-B Kette umfasst die folgenden Sequenzen SEQ ID No. 4, einschließlich deren Isoformen oder ein funktionelles Fragment davon.

### (Nach- und vorstehend allesamt "rekombinante Mistellektine")

Weiterhin bevorzugt ist ein erfindungsgemäßes rekombinantes Mistellektin ein Heterodimer bestehend aus den Sequenzen SEQ ID No. 1 und SEQ ID No. 4, siehe z.B. EP 0 751 221, so genanntes "Aviscuminum" (siehe Beispiele).

Der Begriff "funktionelles Fragment" definiert im Zusammenhang mit dieser Erfindung Fragmente der genannten Polypeptide, welche die gleiche biologische Funktion besitzen, wie das mit der jeweiligen Aminosäuresequenz oben dargestellte Polypeptid.

Der Begriff "gleiche biologische Funktion" beschreibt in diesem Zusammenhang beispielsweise, dass Fragmente oder Derivate der Polypeptide die gleichen Signale in einer Zelle induzieren, wie die genannten Peptide. Beispiele für Fragmente sind Peptiddomänen mit definierten Funktionen. Die "gleiche biologische Funktion" umfasst auch die Zytotoxizität, Immunstimulation (sowohl des nativen, als auch des adaptiven Immunsystems), Stimulation der Freisetzung von Zytokinen, Antigenität, die Induktion der Expression oder die Aktivierung von Oberflächenmarkern, die Induktion von Apoptose oder Endorphin-Stimulation.

Unter "biologischer Aktivität des rekombinanten Mistellektins" wird hier jede biologische Aktivität aus dem Spektrum der gesamten biologischen Aktivitäten des rekombinanten Mistellektins verstanden. Eine derartige Funktion ist z.B. die pharmakologische Wirkung des rekombinanten Mistellektins, insbesondere die Eignung als Adjuvans in Kombination mit einem Antigen (Impfstoff).

Untersuchungen von ML-I-Monomeren ergaben 25 verschiedene Isoformen, die auf unterschiedliche Kombinationen verschiedener A- und B-Ketten sowie unterschiedliche Glykosylierungszustände der Ketten zurückzuführen sind.

Für die vorliegende Erfindung kommt daher auch ein Mistellektin-Polypeptid oder ein Fragment davon, welches die Sequenzvariabilität der verschiedenen MLA- und MLB-Ketten umfasst, zu den Sequenzen SEQ ID No. 1 und 4 erfindungsgemäß in Betracht.

Das erfindungsgemäße Arzneimittel enthält mindestens ein rekombinantes Mistellektin-Polypeptid mit den Sequenzen SEQ ID No. 1 und 4. Beispielsweise konnte eine erhöhte Immunantwort bei einer Verabreichung von einem erfindungsgemäßen Vakzin mit dem Hepatitis B Oberflächenantigen HBsAg (Hepatitis B surface antigen) als auch dem Ovalbumin als "schwaches Modellantigen" beobachtet werden (siehe Beispiele). Ferner wurde überraschender Weise festgestellt, dass die Zellaktivierung durch rekombinante Mistellektine über eine effiziente und besonders vorteilhafte Aktivierung des Inflammasoms vergleichbar zu der Wirkungsweise von Aluminum (Aluminiumhydroxid) verläuft.

In einer weiteren besonders vorteilhaften Ausführungsform der Erfindung beträgt die zu verabreichende Dosis am Patienten (Mensch oder Säugetier) 5 bis 600 ng/ml, insbesondere 250 bis 450 ng/ml, 350 ng/ml, bzw. 5 bis 600 ng/Patient, insbesondere 250 bis 450 ng/Patient, 350 ng/Patient, der rekombinanten Mistellektine, welche besonders bevorzugt subkutan appliziert wird.

Es konnte in den Beispielen gezeigt werden, dass im Bereich von 5 bis 50 ng/Tier (bzw. 50 bis 500 ng/mL), bzw. am Patienten eine optimale Interleukin-1β (IL-1β) Freisetzung in dem engen Dosisbereich von 280 - 420 ng/Patient (280 - 420 ng/mL), insbesondere nach subkutaner Verabreichung, erfolgt. Bei höheren Dosen findet eine Interleukinfreisetzung nicht statt, sondern vielmehr können zytotoxische Effekte am gesunden Gewebe auftreten. IL-1β ist ein direkter Parameter für eine Inflammasomaktivierung, die für die Eignung als Adjuvans besonders vorteilhafte ist. rekombinanten Mistellektins ist eine vorteilhafte exakte Dosierung in einem engen Dosisbereich möglich ist.

Dies führt ebenfalls zu einer verbesserten Verträglichkeit in der Anwendung, so dass weniger Anwendungen zur erfolgreichen Impfung vonnöten sind. Lavelle (supra) benötigt z.B. mangels ausreichender Inflammasomaktivierung vier Anwendungen für einen Impferfolg. Gemäß Beispiel 2 wird in hinreichender Analogie ein Impferfolg bereits nach 2 Anwendungen erreicht.

Die rekombinanten Mistellektine müssen aufgrund der dargelegten Wirkungsmechanismen der Adjuvans-Kategorie "immune potentiators" (Immunverstärker (supra)) zugerechnet werden.

Die Aktivierung von APCs erfolgt durch:
1. Aktivierung von Phagozyten (dendritische Zellen & Monozyten/Makrophagen) aufgrund von Phagozytose von Aviscuminum mit den Konsequenzen von
   a. Antigenpräsentation
   b. Initiierung von T-Zellantworten und
   c. Zytokinsekretion
   und
2. Induktion von Apoptose (z.B. Monozyten/Makrophagen) mit den Konsequenzen von
   a. Aktivierung von benachbarten Phagozyten durch Aufnahme von toten Zellen (z.B. apoptotische Körper) aus Apoptose und
   b. Zytokinsekretion

Ferner wird die vorteilhafte Inflammasom-Aktivierung in Monozyten mittels den erfindungsgemäßen rekombinanten Mistellektinen durch Caspase-1-Stimulation und Sekretion von IL-1β und IL-18 gezeigt. Mehrere Mechanismen zur Generierung von starken Immunantworten kennzeichnen daher rekombinante Mistellektine als ein starkes und einzigartiges Adjuvans.

Insbesondere erlauben die erfindungsgemäßen rekombinanten Mistellektine eine spezifische und signifikante Inflammasomaktivierung, die erfindungsgemäß wesentlich für die Eignung als Adjuvans angesehen wird.

Daher betrifft die Erfindung ebenfalls die Verwendung von rekombinanten Mistellektinen als Adjuvans in Vakzinen zum Verstärken der Immunantwort eines Wirts (Tier, Säugetier oder Mensch) gegen ein oder mehrere Antigene.

Erfindungsgemäß werden Vakzine bereitgestellt, die mindestens ein rekombinantes Mistellektin - Adjuvans sowie ein Antigen, z.B. ein aus Hefezellen hergestelltes Antigen HBsAg und ggf. einen pharmazeutisch verträglichen Träger oder ein pharmazeutisch verträgliches Verdünnungsmittel, sowie ggf. sonstige Bestandteile, wie beispielsweise Natriumchlorid, Dinatriumphosphatdihydrat, Natriumdihydrogenphosphat enthalten.

Die erfindungsgemäßen Vakzine werden vorzugsweise in einer Phosphatgepufferten Kochsalz-Lösung formuliert und als sterile Suspension zur Injektion in einer Durchstechflasche oder in einer Fertigspritze bereitgestellt und lösen eine immunologische Antwort im Wirt (Tier, Säugetier oder Mensch) oder Proband / Patient aus. Hierbei induzieren die erfindungsgemäßen Vakzine die Bildung spezifischer T-Zellen und die Bildung humoraler Antikörper gegen HBsAg.

Die erfindungsgemäßen Vakzine dienen z.B. zur aktiven Immunisierung gegen Hepatitis-B-Viren, verursacht durch Viren aller bekannten Subtypen bei nichtimmunen Personen aller Altersgruppen. Die zu impfenden Personengruppen sind den offiziellen Impfempfehlungen zu entnehmen (Ständige Impfkommission am Robert-Koch-I nstitut).

Für eine Grundimmunisierung mit einem erfindungsgemäßen Vakzin können folgende Impfschemata dienen:
1. Das Impfschema mit Impfungen im Monat 0, 1, 6 führt zu hohen Antikörperkonzentrationen und zu einem in der Regel optimalen Schutz im Monat 7.
2. Das beschleunigte Impfschema mit Impfungen nach 0, 1 und 2 Monaten ermöglicht einen schnellen Aufbau des Impfschutzes. Nach 12 Monaten sollte eine vierte Dosis gegeben werden, um einen Langzeitschutz zu induzieren.

Diese beispielhaften Impfschemata können gemäß den nationalen Impfempfehlungen (Ständige Impfkommission am Robert-Koch-Institut) angepasst werden.

Die erfindungsgemäßen Vakzine werden vorzugsweise subkutan oder intramuskulär verabreicht.

Die Menge eines oder mehrerer Antigene und eines rekombinantes Mistellektin - Adjuvans in den erfindungsgemäßen Vakzinen und die verabreichten Dosen kann durch Techniken bestimmt werden, die dem Fachmann auf dem medizinischen Gebiet bekannt sind. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Vorzugsweise sollte das erfindungsgemäße rekombinante Mistellektin - Adjuvans in den erfindungsgemäßen Vakzinen als eine wässrige phosphatgepufferte Kochsalzlösung verwendet werden und das Antigen ist in der Regel je nach Einheit in einer Größenordnung von Nanogramm, Mikrogramm bis Milligramm vorhanden (vgl. z.B. nachstehende Beispiele).

Das erfindungsgemäße rekombinante Mistellektin - Adjuvans kann mit jedem Antigen von Interesse verwendet werden, so dass ein erfindungsgemäßes Vakzin bereit gestellt wird.

Geeignete Antigene sind vorzugsweise solche die als potentielle Impfstoffe eingesetzt werden und können nicht abschließend solche sein, wie z.B. HBsAg (supra), Antigene gegen Influenzaviren H1N1 und andere Subtypen, Peptidantigene gerichtet gegen immunogene Tumore, beispielsweise PAX- Peptide (Rodeberg DA, Nuss RA, Elsawa SF et al. 2006 Int J Cancer 119: 126-132, Yan M, Himoudi N, Pule M et al. 2008 Cancer Res 68(19): 8058 -8065) u.v.a.

Eine bevorzugte Dosierung eines erfindungsgemäßen Adjuvans ist eine Konzentration von 5 ng und mehr.

Erfindungsgemäße Vakzine werden zweckmäßig als flüssige Zubereitungen oder Zusammensetzungen bereitgestellt, die auf einen ausgewählten pH-Wert gepuffert sein können (supra).

Die Wahl von geeigneten Trägern und anderen Additiven hängt von dem gewünschten Verabreichungsweg und der Art der spezifischen Dosierungsform ab. Ein pharmazeutisch verträgliches Konservierungsmittel kann verwendet werden, um die Lagerungsfähigkeit der Vakzine zu erhöhen.

Die erfindungsgemäßen Vakzine werden dadurch hergestellt, dass die Bestandteile unter Befolgung allgemein akzeptierter Verfahren gemischt oder einzeln verabreicht werden.

Die nachstehenden Beispiele dienen zur Veranschaulichung der Erfindung, ohne die Erfindung auf diese Beispiele zu beschränken.

### Beispiele

### Beispiel 1: Aktivierung des Inflammasoms durch Aviscuminum

PBMCs, isoliert durch FICOLL - Gradientenaufreinigung aus humanen Blut von Spendern, werden in Titerplatten ausgesät und mit Avicusminum 8 bis 24 Stunden mit oder ohne LPS Kostimulation inkubiert. LPS aktiviert über den Toll-like Rezeptor 4 (TLR4) und nachfolgender Caspasel Aktivierung das pro-IL-1β und pro-IL-18, ohne das es zur Ausschüttung der Zytokine durch LPS allein kommt.
In der Kokultur von Aviscuminum und LPS werden die Zytokine IL-1β und IL-18 konzentrationsabhängig freigesetzt. Eine Sekretion von IL-6 und TNF-alpha wird nicht induziert. (9 Stunden Experiment). Aviscuminum und LPS jeweils allein in der Zellkultur führen nicht zur Freisetzung der Zytokine IL-1β. Aviscuminum allein ist in der Lage IL-18 freizusetzen, dieser Effekt wird durch LPS verstärkt.. Werden die PBMCs über magnetische Zellsortierung (anti-CD14 MACS-beads, Miltenyi) von den Monozyten befreit, kann in den verbleibenden PBMCs durch Inkubation mit Aviscuminum und LPS kein IL-1β und IL-18 freigesetzt werden. Folglich erfolgt die Freisetzung der Zytokine IL-1β und Zellen IL-18 aus den Monozyten.
Die Induktion von IL-1β und IL-18 durch die gemeinsame Inkubation der PBMCs mit Aviscuminum und LPS zeigt die Aktivierung des Inflammasom Komplexes an.

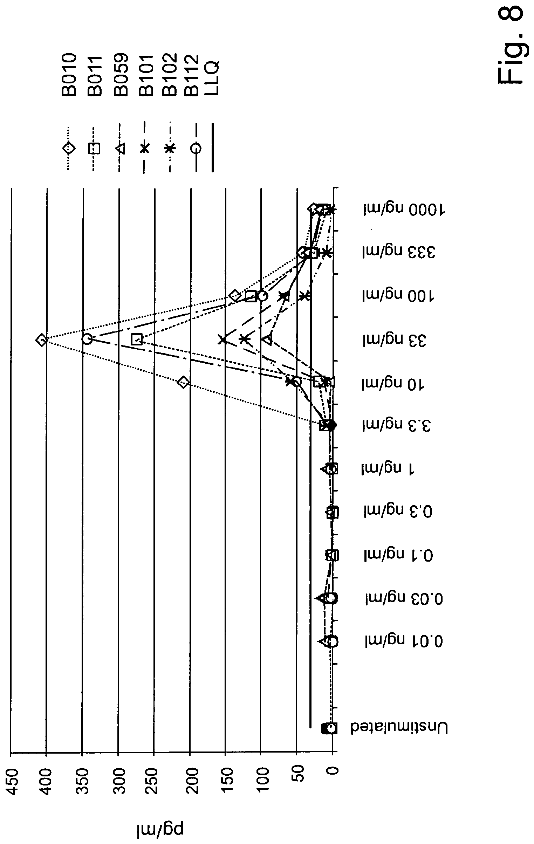

In-vitro wird eine optimale IL-1β Freisetzung aus humanen peripheren monozytären Blutzellen (PBMC) je nach Dauer der Inkubation mit Aviscuminum in Abwesenheit von LPS in einem engen Konzentrationsbereich bei einer optimalen Konzentration von 33 ng/mL gesehen. Dieser Effekt folgt einer glockenförmigen KonzentrationsWirkungsbeziehung (Figur 8).

### a.) Immunisierungen

Es werden zwei Immunisierungen an einem bestimmten Inzuchtmäusestamm (BALB/c-Linie) durchgeführt, wobei Aviscuminum als Adjuvans und das Hepatitis B-Oberflächenantigen (HBsAg) und in einem weiteren Experiment Ovalbumin als Antigen zusammen verabreicht wird. Die Immunisierungen werden im Abstand von 3 Wochen durchgeführt. Die Antigen-Aviscuminum-Formulierungen werden subkutan an Tag 1 und Tag 21 verabreicht und die immunisierten Tiere werden 14 Tage nach der letzten Immunisierung getötet.
Zur Bestimmung der Immunogenität werden eine intrazelluäre Zytokinfärbung von antigenspezifischen CD8 und CD4 T-Zellen und eine Bestimmung der antigenspezifischen Antikörper durchgeführt. Diese Versuchsanordnung ermöglicht den Nachweis einer verstärkten Immunantwort aufgrund von Aviscuminum auf zellulärer und humoraler Ebene.

### Beispiel 2: Immunisierung mit Hepatitis B Oberflächenantigen (HBsAg)

Das Antigen HBsAg ist ein Lipoproteinpartikel mit einer Größe von 20 nm. Solch ein virus-ähnliches Partikel ist in der Lage eine humorale Immunantwort hervorzurufen, manchmal sogar in Abwesenheit eines Adjuvans. Die Stimulierung von geringen HBsAg CD8 T-Zell-Antworten ist bei BALB/c-Mäusen allein durch das Antigen möglich.

Diese Kombination aus Antigen und Mausstamm ermöglicht die Analyse der Fähigkeit von Aviscuminum geringe humorale und zelluläre Antworten zu verstärken. Dabei wird AbISCO^{®}-100 (ISCONOVA, Uppsala, Schweden) als Positivkontrolle verwendet, da bekannt ist, dass dieses Adjuvans die Immunantwort in diesem Modell erhöht.
Mausstamm: BALB/C
2 Immunisierungen subkutan (Tage 1, 21)
5 Tiere pro Gruppe (3 Tiere für die Kontrollgruppen 2 und 3); 31 Tiere insgesamt

**Tabelle 1:**

| Gruppe | HBsAg (Dosis/Tier) | Adjuvans (Dosis/Tier) | Bemerkung |
|---|---|---|---|
| 1/A | 5 µg | AbISCO Adjuvans | Positivkontrolle |
| 2/B | - | - | PBS Negativkontrolle |
| 3/C | - | 50 ng Aviscuminum | Adjuvants-Kontrolle |
| 4/D | 5 µg | - | Antigen-Kontrolle |
| 5/E | 5 µg | 0,5 ng Aviscuminum | geringe Dosis |
| 6/F | 5 µg | 5 ng Aviscuminum | mittlere Dosis |
| 7/G | 5 µg | 50 ng Aviscuminum | hohe Dosis |

Die Impfstoffformulierungen werden vor jeder Immunisierung frisch hergestellt. Aviscuminum wird in PBS w/0,01% Tween 80 verdünnt.

### Reagenzien

5µg rekombinantes HBsAg (Rhein Biotech GmbH, Düsseldorf Deutschland) pro Tier wird als Antigen für Formulierungen (100µl Volumen pro Dosis / Tier) verwendet.

Als Kontrolltest wird das Adjuvans AbISCO-100 (Isconova, Uppsala, Sweden) mit 12 µg pro Dosis verwendet. AbISCO-100 ist ein Adjuvans, welches für die Verwendung in Mäusen optimiert worden ist.

Zur Restimulierung von CD8+ Zellen werden für BALB/c-Mäuse die folgenden synthetischen Peptide verwendet: HBsAg₂₈₋₃₉ IPQSLDSWWTSL als MHC Klasse I (L^{d})-restringierte HBsAg-spezifische Peptide. Malaria CSP₂₈₀₋₂₈₉ SYVPSAEQI als MHC Klasse I (K^{d})-restringierte irrelevante Kontrollpeptide.

CD4+ spezifische T-Zellen werden mit HBsAg-Antigenen restimuliert (verwendet wie zur Impfstoffformulierung).

### Untersuchungen

### a.) Bestimmung von HBsAg spezifischen IFNγ produzierenden CD8+ T-Zellen aus der Milz

Am Tag 14 nach der zweiten Immunisierung wird eine Einzelzellsuspension der Milz ex vivo für 4 Stunden mit den entsprechenden antigenspezifischen Peptiden restimuliert. Brefeldin A wird zugegeben, um die produzierten Zytokine intrazellulär zuhalten. Die Zelloberflächen werden gegen CD8 gefärbt, fixiert und für nachfolgende intrazelluläre IFN-γ-Färbung permeabilisiert. Gefärbte Zellen werden mit einem Beckmann Coulter Durchflusszytometer (FC 500) mit CXP-Software analysiert. 60 000 CD8 positive Zellen werden analysiert. Die Anzahl von CD8+IFN-γ+ T-Zellen pro 10⁵ CD8+ T-Zellen wird angegeben.

### b.) Bestimmung von HBsAg spezifischen IFNγ produzierenden CD4+ T-Zellen aus der Milz

Am Tag 14 nach der zweiten Immunisierung wird eine Einzelzellsuspension der Milz ex vivo über Nacht mit 1 µg/mL rekombinantem HBsAg restimuliert. Das Medium zur Restimulation wird als Negativkontrolle verwendet. Nach 4 Stunden Behandlung mit Brefeldin A werden die Zelloberflächen gegen CD4 gefärbt, fixiert und für nachfolgende intrazelluläre IFN-γ-Färbung permeabilisiert. Die Zellen werden mit einem Beckmann Coulter Durchflusszytometer (FC 500) mit CXP-Software analysiert. 60 000 CD4 positive Zellen werden analysiert. Die Anzahl von CD4+IFN-γ+ T-Zellen pro 10⁵ CD4+ T-Zellen wird angegeben.

### c.) Quantifizierung von spezifischen HBV-Oberflächen-Antikörpern im Maus-Serum

Zur Quantifizierung von anti-HBsAg-Antikörpern wird IMx AUSAB (ELISA) mit dem IMx Reader (Abbott Diagnostics) verwendet. Der Test wird entsprechend der Herstellerangaben durchgeführt.

### d.) Bestimmung des T-Helferzell (Th)-Profils der Immunantwort

Antigenspezifische IgG1- und IgG2b-Titer werden durch ELISA bestimmt und das IgG1/IgG2b-Verhältnis wird zur Beurteilung des Th-Profils verwendet.

### Statistik

Zur statistischen Analyse der Unterschiede zwischen zwei Gruppen wird der T-Test verwendet (GraphPad Prism 5 Software).

### Ergebnisse

### HBsAg spezifische T-Zellantwort:

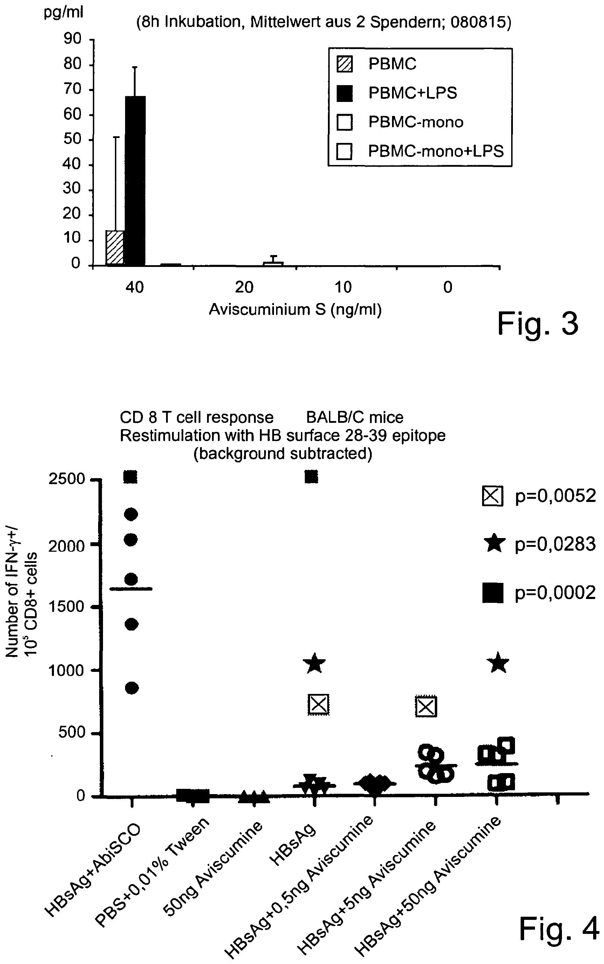

Nach 2 Immunisierungen wurde eine erhöhte HBsAg-spezifische CD8+ T-Zellantwort bei den 50 ng und 5 ng Aviscuminum-Gruppen festgestellt (siehe Figur 4).

Die Gruppe, welche HBsAg mit 0,5 ng Aviscuminum aufweist, zeigte keine verstärkte CD8+ Antwort, verglichen zu HBsAg alleine (welche eine gewisse CD8+ Antwort in BALB/c Mäusen aufweist). Die Positivkontrolle AbISCO zeigte, wie erwartet, eine wirksame CD8+ Antwort. Es wurde in Puffer und Adjuvans-Kontrollgruppen kein Hintergrund beobachtet (siehe Figur 4).

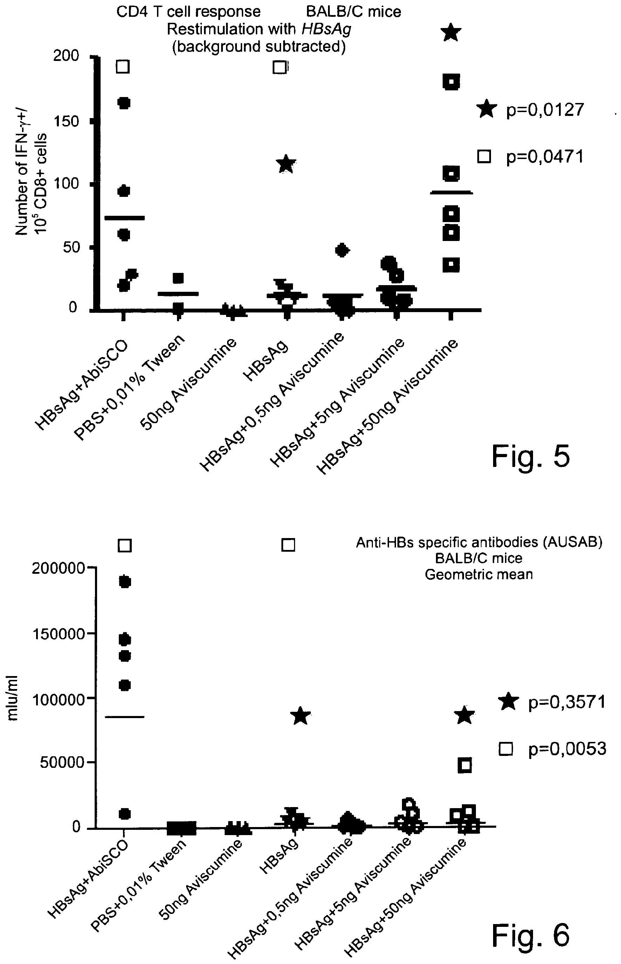

Eine HBsAg-spezifische CD4 T-Zellantwort wurde in der Gruppe beobachtet, welche HBsAg + 50 ng Aviscuminum erhalten hatte (siehe Figur 5).
Die Stärke dieser CD4+ Antwort ist vergleichbar mit der, welche mit AbISCO erzielt worden ist. Geringere Dosen von Aviscuminum als auch Puffer und AdjuvansKontrolle zeigte keine HBsAg-spezifische CD4-Antwort (siehe Figur 5).

### Anti-HBsAg Antikörperantwort

Eine schwache Anti-HBs-Antikörperantwort wurde 2 Wochen nach der zweiten Immunisierung in der 50 ng Aviscuminum-Gruppe festgestellt (siehe Figur 6).

In BALB/c Mäusen zeigt sich bei wiederholter Immunisierung mit HBsAg alleine schon eine schwache anti-HBs-Antikörperantwort.

Die Positivkontrolle AbISCO induziert, wie erwartet, eine starke Antikörperantwort, während Puffer und Adjuvanskontrolle negativ sind.

Das T-Helferzell-Profil wurde für HBsAg immunisierte Tiere wegen geringer oder negativer anti-HBs-Titer nicht bestimmt.

### Beispiel 3: Immunisierung mit Ovalbumin (OVA)

Ovalbumin (OVA) ist ein lösliches, monomeres Protein, welches nur in Verbindung mit Adjuvantien anti-OVA Antikörper induzieren kann. Es wird AbISCO^{®}-100 (ISCONOVA, Uppsala, Schweden) als Positivkontrolle verwendet, da bekannt ist, dass dieses Adjuvans die Immunantwort in diesem Model erhöht.
Mausstamm: C57BL/6
2 Immunisierungen s.c. (Tag 1, 21)
5 Tiere pro Gruppe (3 Tiere für die Kontrollgruppen 9 und 10); 31 Tiere insgesamt

| Gruppe | OVA (Dosis/Tier) | Adjuvans (Dosis/Tier) | Bemerkung |
|---|---|---|---|
| 8/H | 10 µg | AbISCO Adjuvans | Positivkontrolle |
| 9/ I | - | - | PBS Negativkontrolle |
| 10/K | - | 50 ng Aviscuminum | Adjuvanskontrolle |
| 11/L | 10 µg | - | Antigenkontrolle |
| 12/M | 10 µg | 0.5 ng Aviscuminum | niedrige Dosis |
| 13/N | 10 µg | 5 ng Aviscuminum | mittlere Dosis |
| 14/O | 10 µg | 50 ng Aviscuminum | hohe Dosis |

Die Impfstoffformulierungen werden vor jeder Immunisierung frisch hergestellt. Aviscuminum wird in PBS w/0,01% Tween 80 verdünnt.

### Reagenzien

10µg Ovalbumin, Endotoxin arm (Hyglos GmbH, Regensburg Deutschland) pro Tier wird als Antigen für Formulierungen (100µl Volumen pro Dosis / Tier) verwendet.

Als Kontrolltest wird das Adjuvans AbISCO-100 (Isconova, Uppsala, Sweden) mit 12 µg pro Dosis verwendet. AbISCO-100 ist ein Adjuvans, welches für die Verwendung in Mäusen optimiert worden ist.

### Untersuchungen

### a.) Quantifizierung von spezifischen OVA-Antikörpern im Maus-Serum Alpha Diagnostics International

Zur Quantifizierung von anti-OVA-Antikörpern wird der Maus anti-OVA ELISA von Alpha Diagnostics International verwendet. Der Test wird entsprechend der Herstellerangaben durchgeführt.

### b.) Bestimmung des T-Helferzell (Th)-Profils der Immunantwort

Antigenspezifische IgG1- und IgG2b-Titer werden durch ELISA bestimmt und das IgG1/IgG2b-Verhältnis wird zur Beurteilung des Th-Profils verwendet.

### Statistik

Zur statistischen Analyse der Unterschiede zwischen zwei Gruppen wird der T-Test verwendet (GraphPad Prism 5 Software).

### Ergebnisse

### anti-OVA Antikörper

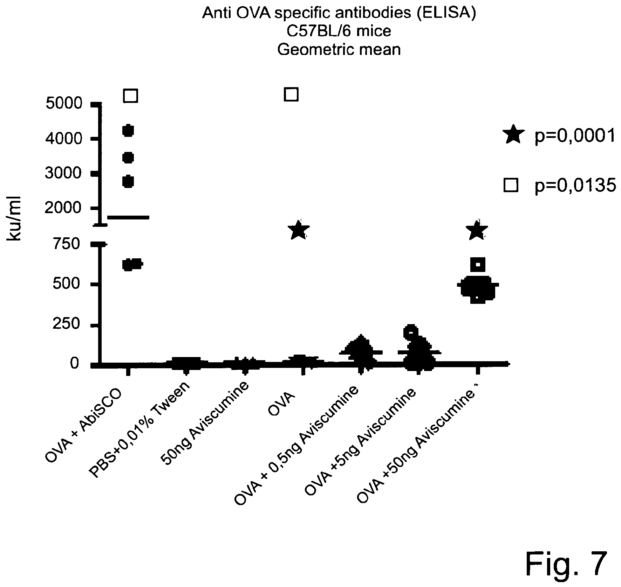

Eine OVA-spezifische Antikörperantwort wurde in der Gruppe mit 50 ng Aviscuminum beobachtet (siehe Figur 7). Puffer und die Adjuvanskontrollgruppen waren negativ. Die AbISCO-Kontrollgruppe war positiv (siehe Figur 7).

Die Bestimmung der OVA-spezifischen IgG1- und IgG2b-Isotypen in den höchsten Aviscuminum-Gruppen (50 ng) zeigte, dass die anti-OVA-Antikörper von IgG1 dominiert waren. Dies deutet auf ein Th2-Profil von der Immunantwort hin.

### Allgemeine Bemerkung

Nach der ersten und zweiten Immunisierung wurden keine unerwünschten Ereignisse in den Tieren beobachtet. Alle Tiere waren zum Zeitpunkt der Tötung gesund.

### Fazit

Die Untersuchung von Aviscuminum mit den Antigenen HBsAg und OVA zeigte in Mäusen nach subkutaner Immunisierungen (an Tag 1 und 21) einen adjuvanten Effekt bei höheren verabreichten Dosen (5 (50 ng/mL) und 50 ng (500 ng/mL)).

T-Zellantwort:
Eine CD8+ und eine CD4+ Antwort ist gegen HBsAg zu beobachten, wenn es zusammen mit Aviscuminum (50 ng (500 ng/mL) und 5 ng (50 ng/mL) Dosis) verabreicht wird. Insbesondere wird die CD4+ T-Zellantwort durch Aviscuminum stark unterstützt.

Da HBsAg alleine bekanntermaßen eine gewisse CD8+Antwort in BALB/c Mäusen zeigt, verstärkt der adjuvante Effekt von Aviscuminum diesen Baseline-Level.

Diese Ergebnisse zeigen, dass Aviscuminum adjuvante Eigenschaften hat, insbesondere auf CD4+ T-Helferzell-Antworten.

### Antikörper-Antwort

Eine anti-HBsAg-Antwort war bis auf in einem Tier von 5 Tieren negativ. Es ist bekannt, dass anti-HBsAg-Antikörper eine langsame Kinetik aufweisen. Deshalb ist es wahrscheinlich, dass die Tiere, die keine anti-HBsAg-Antikörper zeigten, zu einem späteren Zeitpunkt positiv geworden wären. Antikörper werden idealerweise 4 Wochen nach der Immunisierung bestimmt, jedoch war der Fokus der Untersuchung auf die T-Zellantwort gerichtet und demnach mussten die Tiere 14 Tage nach der Immunisierung getötet werden.

Eine anti-OVA-Antikörperantwort wurde zum Zeitpunkt der Tötung der Tiere, d.h. 14 Tage nach der zweiten Immunisierung beobachtet.

Die Bestimmung der anti-OVA-Antikörperisotypen zeigte ein Th 2-Profil der Immunantwort. Dies ist in Übereinstimmung mit der beobachteten Induktion von CD4 T-Zellen.

### Zusammenfassung der Ergebnisse

Aviscuminum-Dosen von 50 ng (500ng/mL) und 5 ng (50 ng/mL) am Tier verstärken spezifische CD8- und insbesondere CD 4 T-Zell-Antworten gegen HBsAg, das ein komplexes Lipoprotein-Antigen darstellt. Spezifische Antikörper mit einem Th 2-Profil wurden gegen OVA ermittelt.

Diese Daten zeigen, dass Aviscuminum einen adjuvanten Effekt mit verstärkter CD4 T-Helfer-Zellantwort aufweist.

Die Dosen von Aviscuminum, die eine verstärkte Immunantwort gegen HBsAG zeigten, waren gering.

Der adjuvante Effekt von Aviscuminum kann wahrscheinlich durch Modifikation der Dosen bzw. der Booster Immunisierungen bzw. Depot-Formulierungen verstärkt werden, um in allen Fällen spezifische Antikörper Konzentrationen zu erreichen.

Beschreibung der Figuren:
Figur 1 beschreibt den Wirkmechanismus von nicht-spezifischen Adjuvantien (Figur von Lamine Mbow M et al. 2010 (supra)).
Figur 2 zeigt die Ausschüttung von IL-1β (pg/ml) aus humanen PBMCs durch Aviscuminum und LPS.
Figur 3 zeigt die Ausschüttung von IL-18 (pg/ml) aus humanen PBMCs durch Aviscuminum und durch Aviscuminum und LPS.
Figur 4 zeigt die Ergebnisse der Untersuchung von HBsAg spezifischer T-Zellantwort, wobei die CD8 T-Zellantwort in BALB/c-Mäusen gemessen wurde. Restimulation erfolgte mit HB-Oberflächenepitop 28-39.
Figur 5 zeigt die Ergebnisse der Untersuchung von HBsAg spezifischer T-Zellantwort, wobei die CD4 T-Zellantwort in BALB/c-Mäusen gemessen wurde. Restimulation erfolgte mit HBsAg.
Figur 6 zeigt die Ergebnisse der Untersuchung von Anti-HBs-Antikörperantwort in BALB/c-Mäusen.
Figur 7 zeigt die Ergebnisse der Untersuchung von Anti-OVA-Antikörperantwort in C57BL/6-Mäusen.
Figur 8 zeigt die Aviscuminum induzierte IL-1β Ausschüttung (pg/mL) aus peripheren monozytären Blutzellen (PBMC) freiwilliger Spender nach einer 24 Stunden Inkubation in Abwesenheit von LPS.

## Patentansprüche

1. Vakzine enthaltend ein oder mehrere Antigene und mindestens ein Adjuvans ausgewählt aus einem rekombinanten Mistellektin mit den Aminosäuresequenzen SEQ ID No. 1 und SEQ ID No. 4, wobei das rekombinante Mistellektin keine Glykolisierung aufweist.

2. Arzneimittel oder Zusammensetzung enthaltend ein oder mehrere Antigene und mindestens ein Adjuvans ausgewählt aus einem rekombinanten Mistellektin mit den Aminosäuresequenzen SEQ ID No. 1 und SEQ ID No. 4 zur Verwendung als Vakzin, und das rekombinante Mistellektin keine Glykolisierung aufweist.

3. Arzneimittel oder Zusammensetzung nach Anspruch 2, wobei das Adjuvans in einer Konzentration 5 bis 600 ng/ml, insbesondere 250 bis 450 ng/ml, 350 ng/ml, bzw. 5 bis 600 ng/Patient, insbesondere 250 bis 450 ng/Patient, 350 ng/Patient vorliegt.

4. Arzneimittel oder Vakzin nach einem der vorhergehenden Ansprüche, wobei diese subkutan oder intramuskulär verabreicht werden.

5. Arzneimittel oder Vakzin nach einem der vorhergehenden Ansprüche wobei das rekombinante Mistellektin-Polypeptid eine Mistellektin-A-Kette ist, ausgewählt aus der Aminosäuresequenz SEQ ID No. 1.

6. Arzneimittel oder Vakzin nach einem der vorhergehenden Ansprüche, wobei das rekombinante Mistellektin-Polypeptid eine Mistellektin-B-Kette ist, ausgewählt aus den Aminosäuresequenzen SEQ ID No. 4.

7. Arzneimittel oder Vakzin nach einem der vorhergehenden Ansprüche, wobei die Vakzine zusätzlich einen pharmazeutisch verträglichen Träger oder ein pharmazeutisch verträgliches Verdünnungsmittel enthält, ggfs. Hilfs- und Zusatzstoffe.

8. Arzneimittel oder Zusammensetzung enthaltend ein Vakzin nach Anspruch 1.

## Claims

1. A vaccine containing one or more antigens and at least one adjuvant selected from a recombinant mistletoe lectin with the amino acid sequences SEQ ID No. 1 and SEQ ID No. 4, wherein the recombinant mistletoe lectin does not have any glycosylation.

2. A medicament or composition containing one or more antigens and at least one adjuvant selected from a recombinant mistletoe lectin with the amino acid sequences SEQ ID No. 1 and SEQ ID No. 4 for use as a vaccine, the recombinant mistletoe lectin having no glycosylation.

3. The medicament or composition according to claim 2, wherein the adjuvant is present in a concentration of 5 to 600 ng/ml, in particular 250 to 450 ng/ml, 350 ng/ml, or 5 to 600 ng/patient, in particular 250 to 450 ng/patient, or 350 ng/patient.

4. The medicament or vaccine according to any one of the preceding claims, wherein these are administered subcutaneously or intramuscularly.

5. The medicament or vaccine according to any one of the preceding claims, wherein the recombinant mistletoe lectin polypeptide is a mistletoe lectin A chain, selected from the amino acid sequence SEQ ID No. 1.

6. The medicament or vaccine according to any one of the preceding claims, wherein the recombinant mistletoe lectin polypeptide is a mistletoe B chain, selected from the amino acid sequences SEQ ID No. 4.

7. The medicament or vaccine according to any one of the preceding claims, wherein the vaccine additionally contains a pharmaceutically acceptable carrier or a pharmaceutically acceptable diluent, and optionally auxiliaries and additives.

8. A medicament or composition containing a vaccine according to claim 1.

## Revendications

1. Vaccins contenant un ou plusieurs antigènes et au moins un adjuvant choisi parmi une lectine de gui de recombinaison avec les séquences d'acides aminés SEQ ID N ° 1 et SEQ ID N° 4, où la lectine de gui de recombinaison ne présente pas de glycolisation.

2. Produit pharmaceutique ou composition contenant un ou plusieurs antigènes et au moins un adjuvant choisi parmi une lectine de gui de recombinaison avec les séquences d'acides aminés SEQ ID N ° 1 et SEQ ID N ° 4 pour l'utilisation en tant que vaccin, et la lectine de gui de recombinaison ne présente pas de glycolisation.

3. Produit pharmaceutique ou composition selon la revendication 2, dans lequel l'adjuvant est présent dans une concentration de 5 à 600 ng/ml, notamment de 250 à 450 ng/ml, 350 ng/ml, c'est-à-dire 5 à 600 ng/patient, notamment 250 à 450 ng/patient, 350 ng/patient.

4. Produit pharmaceutique ou vaccin selon l'une des revendications précédentes, où celui-ci est administré par voie sous cutanée ou intramusculaire.

5. Produit pharmaceutique ou vaccin selon l'une des revendications précédentes, où le polypeptide de lectine de gui de recombinaison est une chaîne A de lectine de gui, choisie dans la séquence d'acides aminés SEQ ID N°1.

6. Produit pharmaceutique ou vaccin selon l'une des revendications précédentes, où le polypeptide de lectine de gui de recombinaison est une chaîne B de lectine de gui, choisie dans la séquence d'acides aminés SEQ ID N°4.

7. Produit pharmaceutique ou vaccin selon l'une des revendications précédentes, où les vaccins contiennent en outre un support pharmaceutiquement acceptable ou un produit de dilution pharmaceutiquement acceptable, éventuellement, des substances auxiliaires et supplémentaires.

8. Produit pharmaceutique ou composition contenant un vaccin selon la revendication 1.


### SEQUENCE LISTING

<110> Cytavis Biopharma GmbH
<120> Rekombinantes Mistellektine und dessen Verwendung als Adjuvans
<130> CYT26WO
<160> 12
<170> PatentIn version 3.3
<210> 1
   <211> 253
   <212> PRT
   <213> Artificial
<220>
   <223> EP0751221 recombinant Protein
<220>
   <221> misc_feature
   <222> (1)..(1)
   <223> Xaa can be Met or can be deleted
<220>
   <221> misc_feature
   <222> (5)..(5)
   <223> Xaa can be Ile or Neu
<220>
   <221> misc_feature
   <222> (16)..(16)
   <223> Xaa can be Glu or Asp
<400> 1
   Xaa Tyr Glu Arg Xaa Arg Leu Arg Val Thr His Gln Thr Thr Gly Xaa
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Gly
       50                  55                  60
   Asp Ser Ile Thr Ala Ala Ile Asp Val Thr Asn Leu Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Ser Ser Leu Pro Phe
               100                 105                 110
   Asn Gly Ser Tyr Pro Asp Leu Glu Arg Tyr Ala Gly His Arg Asp Gln
           115                 120                 125
   Ile Pro Leu Gly Ile Asp Gln Leu Ile Gln Ser Val Thr Ala Leu Arg
       130                 135                 140
   Phe Pro Gly Gly Ser Thr Arg Thr Gln Ala Arg Ser Ile Leu Ile Leu
   145                 150                 155                 160
   Ile Gln Met Ile Ser Glu Ala Ala Arg Phe Asn Pro Ile Leu Trp Arg
                   165                 170                 175
   Ala Arg Gln Tyr Ile Asn Ser Gly Ala Ser Phe Leu Pro Asp Val Tyr
               180                 185                 190
   Met Leu Glu Leu Glu Thr Ser Trp Gly Gln Gln Ser Thr Gln Val Gln
           195                 200                 205
   His Ser Thr Asp Gly Val Phe Asn Asn Pro Ile Arg Leu Ala Ile Pro
       210                 215                 220
   Pro Gly Asn Phe Val Thr Leu Thr Asn Val Arg Asp Val Ile Ala Ser
   225                 230                 235                 240
   Leu Ala Ile Met Leu Phe Val Cys Gly Glu Arg Pro Ser
                   245                 250
<210> 2
   <211> 256
   <212> PRT
   <213> Artificial
<220>
   <223> EP1051495 recombinant Protein
<220>
   <221> misc_feature
   <222> (1)..(1)
   <223> Xaa can be Met or can be deleted
<220>
   <221> misc_feature
   <222> (16)..(16)
   <223> Xaa can be Asp or Glu
<220>
   <221> misc_feature
   <222> (64)..(64)
   <223> Xaa can be Gly or Gln
<220>
   <221> misc_feature
   <222> (67)..(67)
   <223> Xaa can be Ile or Val
<220>
   <221> misc_feature
   <222> (76)..(76)
   <223> Xaa can be Leu or Ala
<220>
   <221> misc_feature
   <222> (108)..(108)
   <223> Xaa can be Asp-Arg or can be deleted
<220>
   <221> misc_feature
   <222> (114)..(114)
   <223> Xaa can be Asn or Thr
<220>
   <221> misc_feature
   <222> (118)..(118)
   <223> Xaa can be Pro or Thr
<220>
   <221> misc_feature
   <222> (135)..(135)
   <223> Xaa can be Asp or Glu
<220>
   <221> misc_feature
   <222> (142)..(142)
   <223> Xaa can be Ser or Thr
<220>
   <221> misc_feature
   <222> (146)..(146)
   <223> Xaa can be Phe or Tyr
<220>
   <221> misc_feature
   <222> (153)..(153)
   <223> Xaa can be Ala or Thr
<220>
   <221> misc_feature
   <222> (178)..(178)
   <223> Xaa can be Ala or Tyr
<220>
   <221> misc_feature
   <222> (181)..(181)
   <223> Xaa can be Tyr or Asp
<220>
   <221> misc_feature
   <222> (186)..(186)
   <223> Xaa can be Ala or Glu
<220>
   <221> misc_feature
   <222> (192)..(192)
   <223> Xaa can be Val or Met
<220>
   <221> misc_feature
   <222> (220)..(220)
   <223> Xaa can be Ile or Phe
<220>
   <221> misc_feature
   <222> (225)..(226)
   <223> Xaa can be Pro-Ser or Pro-Thr
<220>
   <221> misc_feature
   <222> (233)..(233)
   <223> Xaa can be Thr or Ser
<220>
   <221> misc_feature
   <222> (237)..(237)
   <223> Xaa can be Asp or Ser
<220>
   <221> misc_feature
   <222> (255)..(256)
   <223> Xaa can be Ser-Ser or can be deleted
<400> 2
   Xaa Tyr Glu Arg Leu Arg Leu Arg Val Thr His Gln Thr Thr Gly Xaa
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Xaa
       50                  55                  60
   Asp Ser Xaa Thr Ala Ala Ile Asp Val Thr Asn Xaa Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Xaa Ser Ser Leu Pro
               100                 105                 110
   Phe Xaa Gly Ser Tyr Xaa Asp Leu Glu Arg Tyr Ala Gly His Arg Asp
           115                 120                 125
   Gln Ile Pro Leu Gly Ile Xaa Gln Leu Ile Gln Ser Val Xaa Ala Leu
       130                 135                 140
   Arg Xaa Pro Gly Gly Ser Thr Arg Xaa Gln Ala Arg Ser Ile Leu Ile
   145                 150                 155                 160
   Leu Ile Gln Met Ile Ser Glu Ala Ala Arg Phe Asn Pro Ile Leu Trp
                   165                 170                 175
   Arg Xaa Arg Gln Xaa Ile Asn Ser Gly Xaa Ser Phe Leu Pro Asp Xaa
               180                 185                 190
   Tyr Met Leu Glu Leu Glu Thr Ser Trp Gly Gln Gln Ser Thr Gln Val
           195                 200                 205
   Gln His Ser Thr Asp Gly Val Phe Asn Asn Pro Xaa Arg Leu Ala Ile
       210                 215                 220
   Xaa Xaa Gly Asn Phe Val Thr Leu Xaa Asn Val Arg Xaa Val Ile Ala
   225                 230                 235                 240
   Ser Leu Ala Ile Met Leu Phe Val Cys Gly Glu Arg Pro Ser Xaa Xaa
                   245                 250                 255
<210> 3
   <211> 257
   <212> PRT
   <213> Artificial
<220>
   <223> EP1051495 recombinant Protein
<220>
   <221> misc_feature
   <222> (1)..(1)
   <223> Xaa can be Met or can be deleted
<400> 3
   Xaa Tyr Glu Arg Leu Arg Leu Arg Val Thr His Gln Thr Thr Gly Asp
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Gln
       50                  55                  60
   Asp Ser Ile Thr Ala Ala Ile Asp Val Thr Asn Ala Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Asp Arg Ser Ser Leu
               100                 105                 110
   Pro Phe Thr Gly Ser Tyr Thr Asp Leu Glu Arg Tyr Ala Gly His Arg
           115                 120                 125
   Asp Gln Ile Pro Leu Gly Ile Glu Gln Leu Ile Gln Ser Val Ser Ala
       130                 135                 140
   Leu Arg Tyr Pro Gly Gly Ser Thr Arg Ala Gln Ala Arg Ser Ile Leu
   145                 150                 155                 160
   Ile Leu Ile Gln Met Ile Ser Glu Ala Ala Arg Phe Asn Pro Ile Leu
                   165                 170                 175
   Trp Arg Tyr Arg Gln Asp Ile Asn Ser Gly Glu Ser Phe Leu Pro Asp
               180                 185                 190
   Met Tyr Met Leu Glu Leu Glu Thr Ser Trp Gly Gln Gln Ser Thr Gln
           195                 200                 205
   Val Gln His Ser Thr Asp Gly Val Phe Asn Asn Pro Phe Arg Leu Ala
       210                 215                 220
   Ile Ser Thr Gly Asn Phe Val Thr Leu Ser Asn Val Arg Ser Val Ile
   225                 230                 235                 240
   Ala Ser Leu Ala Ile Met Leu Phe Val Cys Gly Glu Arg Pro Ser Ser
                   245                 250                 255
   Ser
<210> 4
   <211> 264
   <212> PRT
   <213> Artificial
<220>
   <223> EP0751221 recombinant Protein
<220>
   <221> misc_feature
   <222> (1)..(1)
   <223> Xaa can be Met or can be deleted
<400> 4
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe Arg Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Leu Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Lys Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro
               260
<210> 5
   <211> 268
   <212> PRT
   <213> Artificial
<220>
   <223> EP0751221 recombinant Protein
<220>
   <221> misc_feature
   <222> (1)..(1)
   <223> Xaa can be Met or can be deleted
<400> 5
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe Arg Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Leu Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Lys Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro Gly Gly Tyr His
               260                 265
<210> 6
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<220>
   <221> misc_feature
   <222> (19)..(19)
   <223> Xaa can be Asn or Ser
<220>
   <221> misc_feature
   <222> (22)..(22)
   <223> Xaa can be Cys or Arg
<220>
   <221> misc_feature
   <222> (57)..(57)
   <223> Xaa can be Gly or Asn
<220>
   <221> misc_feature
   <222> (96)..(96)
   <223> Xaa can be Gly or Asn
<220>
   <221> misc_feature
   <222> (158)..(158)
   <223> Xaa can be Gly or Gln
<220>
   <221> misc_feature
   <222> (167)..(167)
   <223> Xaa can be Val or Asp
<220>
   <221> misc_feature
   <222> (171)..(171)
   <223> Xaa can be Gln or Lys
<220>
   <221> misc_feature
   <222> (174)..(175)
   <223> Xaa can be Gly or can be deleted or can be Gly-Arg or Gly-Lys or Arg
   or Lys
<220>
   <221> misc_feature
   <222> (196)..(196)
   <223> Xaa can be Cys or Val or Ser
<220>
   <221> misc_feature
   <222> (212)..(213)
   <223> Xaa can be Ala-Ala or Ala-Gly or Gly-Ala or Gly-Gly
<220>
   <221> misc_feature
   <222> (215)..(216)
   <223> Xaa can be Ser-Ser or Ser-Gly or Gly-Ser or Gly-Gly
<220>
   <221> misc_feature
   <222> (225)..(225)
   <223> Xaa can be Gly or Tyr
<220>
   <221> misc_feature
   <222> (232)..(236)
   <223> Xaa232 can be Asn, Ser, Thr or Lys, Xaa233 can be Ser or Gly, Xaa234
   can be Leu or Pro, Xaa235 can be Ala or Met, Xaa 236 can be Met or Val
<220>
   <221> misc_feature
   <222> (265)..(265)
   <223> Xaa can be Pro or Phe
<400> 6
   Xaa Asp Asp Val Thr Cys Ser Ala Ser Glu Pro Thr Val Arg Ile Val
   1               5                   10                  15
   Gly Arg Xaa Gly Met Xaa Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Xaa Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Xaa
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Xaa Gly Ser
   145                 150                 155                 160
   Val Trp Val Glu Thr Cys Xaa Ser Ser Gln Xaa Asn Gln Xaa Xaa Trp
                   165                 170                 175
   Ala Leu Tyr Gly Asp Gly Ser Ile Arg Pro Lys Gln Asn Gln Asp Gln
               180                 185                 190
   Cys Leu Thr Xaa Gly Arg Asp Ser Val Ser Thr Val Ile Asn Ile Val
           195                 200                 205
   Ser Cys Ser Xaa Xaa Ser Xaa Xaa Gln Arg Trp Val Phe Thr Asn Glu
       210                 215                 220
   Xaa Ala Ile Leu Asn Leu Lys Xaa Xaa Xaa Xaa Xaa Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Xaa
               260                 265
<210> 7
   <211> 264
   <212> PRT
   <213> Artificial
<220>
   <223> EP1051495 recombinant Protein
<220>
   <221> misc_feature
   <222> (1)..(1)
   <223> Xaa can be Met or can be deleted
<400> 7
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Gln Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro
               260
<210> 8
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 8
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Asn Gly Leu Ala Met Asp Val Ala Gln
   225                 230                 235                 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 9
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 9
   Xaa Asp Asp Val Thr Cys Ser Ala Ser Glu Pro Thr Val Arg Ile Val
   1               5                   10                  15
   Gly Arg Ser Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Asn Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gln Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
   180 185 190
   Cys Leu Thr Val Gly Arg Asp Ser Val Ser Thr Val Ile Asn Ile Val
   195 200 205
   Ser Cys Ser Gly Ala Ser Gly Ser Gln Arg Trp Val Phe Thr Asn Glu
   210 215 220
   Tyr Ala Ile Leu Asn Leu Lys Ser Gly Leu Ala Met Asp Val Ala Gln
   225 230 235 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 10
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 10
   Xaa Asp Asp Val Thr Cys Ser Ala Ser Glu Pro Thr Val Arg Ile Val
   1 5 10 15
   Gly Arg Asn Gly Met Arg Val Asp Val Arg Asp Asp Asp Phe His Asp
   20 25 30
   Gly Asn Gln Ile Gln Leu Trp Pro Ser Lys Ser Asn Asn Asp Pro Asn
   35 40 45
   Gln Leu Trp Thr Ile Lys Arg Asp Gly Thr Ile Arg Ser Asn Gly Ser
   50 55 60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Ile Trp Gln Ile Trp Asp
   85 90 95
   Asn Gly Thr Ile Ile Asn Pro Arg Ser Asn Leu Val Leu Ala Ala Ser
   100 105 110
   Ser Gly Ile Lys Gly Thr Thr Leu Thr Val Gln Thr Leu Asp Tyr Thr
   115 120 125
   Leu Gly Gln Gly Trp Leu Ala Gly Asn Asp Thr Ala Pro Arg Glu Val
   130 135 140
   Thr Ile Tyr Gly Phe Arg Asp Leu Cys Met Glu Ser Asn Gly Gly Ser
   145 150 155 160
   Val Trp Val Glu Thr Cys Asp Ser Ser Gln Lys Asn Gln Gly Lys Trp
   165 170 175
   Ala Leu Tyr Gly Asp Gly Ser Ile Arg Pro Lys Gln Asn Gln Asp Gln
   180 185 190
   Cys Leu Thr Ser Gly Arg Asp Ser Val Ser Thr Val Ile Asn Ile Val
   195 200 205
   Ser Cys Ser Gly Ala Ser Gly Ser Gln Arg Trp Val Phe Thr Asn Glu
   210 215 220
   Gly Ala Ile Leu Asn Leu Lys Thr Gly Leu Ala Met Asp Val Ala Gln
   225 230 235 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 11
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 11
   Xaa Asp Asp Val Thr Cys Ser Ala Ser Glu Pro Thr Val Arg Ile Val
   1 5 10 15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Lys Gly Pro Ala Met Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265
<210> 12
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 12
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Asn Ser Leu Met Val Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265